# EUROPEAN PATENT APPLICATION

(11) **EP 4 318 494 A1**
(43) Date of publication of application: **07.02.2024**
(21) Application number: 22781624.6
(22) Date of filing: 30.03.2022
(51) Int. Cl.: G16H 50/20, G16H 50/50, G16H 50/70, G16H 30/40, G16H 30/20, G06N 3/04, G06N 20/00

(54) **METHOD AND APPARATUS FOR PROVIDING CONFIDENCE INFORMATION ON RESULT OF ARTIFICIAL INTELLIGENCE MODEL**

(30) Priority: 31.03.2021 KR 20210042268; 29.03.2022 KR 20220038924
(71) Applicant: Lunit Inc., Seoul, 06241 (KR)
(72) Inventor: PARK, Sunggyun, Seoul, 05224 (KR); KIM, Kihwan, Hanam-si, Gyeonggi-do, 12917 (KR); PAEK, Seungwook, Seoul, 01913 (KR); YOO, Donggeun, Seoul, 05553 (KR)
(74) Representative: Frenkel, Matthias Alexander
(86) International application number: PCT/KR2022/004544
(87) International publication number: WO 2022/211505

(57) **Abstract**

A computing apparatus operated by at least one processor includes a target artificial intelligence model configured to learn at least one task, and perform a task for an input medical image to output a target result, and a confidence prediction model configured to obtain at least one impact factor that affects the target result based on the input medical image, and estimate confidence information for the target result based on the impact factor.

## Description

### [Technical Field]

The present disclosure relates to an artificial intelligence-based prediction technology.

### [Background Art]

Machine-learning technologies outperform existing methods when analyzing various types of data, such as video, voice, and text. In addition, the scalability and flexibility inherent in the machine-learning technologies lead to their applications in a wide range of fields. Consequently, many different types of neural networks are being released.

Artificial intelligence (AI) technologies based on machine learning are being actively adopted in medical fields. While traditional computer-aided detection (CAD) devices have detected lesions based on predefined rules, or detected lesions in candidate areas of the medical image, recent AI-based medical image reading technologies can analyze the entire image using AI algorithms and visually provide abnormal lesions.

By implementing AI-based medical image reading technology in diagnostic assistant devices, medical staffs can receive information about abnormal lesions detected in medical images, and diagnose the lesions based on the received information.

### [Disclosure]

### [Technical Problem]

The present disclosure provides a method and an apparatus for providing confidence information for a result of an artificial intelligence model using at least one impact factor that affects the result of the artificial intelligence model.

### [Technical Solution]

Some embodiments provide a computing apparatus operated by at least one processor, the computing apparatus including a target artificial intelligence model configured to learn at least one task, and perform a task for an input medical image to output a target result, and a confidence prediction model configured to obtain at least one impact factor that affects the target result based on the input medical image, and estimate confidence information for the target result based on the impact factor.

The at least one impact factor may be determined based on a characteristic of the target artificial intelligence model and/or a characteristic of the input medical image.

The at least one impact factor may include at least one of a task-related medical factor of the target artificial intelligence model, an input image-related factor of the target artificial intelligence model, a disease-related factor detected by the target artificial intelligence model, a patient-related demographic factor, and a patient characteristic-related factor.

The at least one impact factor may be extracted from additional information of the input medical image, inferred from the input medical image, obtained from an external server or database, or input from a user.

The impact factor extracted from the additional information of the input medical image may include at least one of age, gender, or imaging details including an imaging method. The impact factor inferred from the input medical image may include at least one of tissue density, presence of an object in the input medical image, a lesion type, and a change in lesion size.

The target artificial intelligence model may include a model trained to detect a lesion from a medical image or to infer medical diagnostic information or treatment information.

The confidence prediction model may include a model that has learned a relationship between at least one impact factor associated with a medical image for training and a confidence score for a target result inferred from the medical image for training.

The confidence information for the target result may be provided together with the target result, used for correction of the target result, used as an indicator for recommending to retake the input medical image and/or for recommending an imaging method, or used as an indicator for discarding the target result output from the target artificial intelligence model.

Some embodiments provide a method of operating a computing apparatus operated by at least one processor, the method including: receiving a medical image input to a target artificial intelligence model; obtaining, based on the medical image, at least one impact factor that affects a target result output from the target artificial intelligence model; and estimating confidence information for the target result based on the at least one impact factor.

The obtaining the at least one impact factor may include extracting the at least one impact factor from additional information of the medical image, inferring the at least one impact factor from the medical image, obtaining the at least one impact factor from an external server/database, or receiving the at least one impact factor from a user.

The at least one impact factor extracted from the additional information of the medical image may include at least one of age, gender, and imaging details including an imaging method. The at least one impact factor inferred from the input medical image may include at least one of tissue density, presence of an object in the input medical image, a lesion type, and a change in lesion size.

The obtaining the at least one impact factor may include in response to the medical image being a mammogram image, determining density inferred from the mammogram image as the at least one impact factor, or in response to the medical image being a chest X-ray image, determining posterior anterior (PA) or anterior posterior (AP) information extracted from additional information of the chest X-ray image as the at least one impact factor.

The method may further include correcting confidence information for the target result based on the target result, and providing the corrected confidence information as final confidence information for the target result.

The method may further include providing the confidence information together with the target result.

The method may further include correcting the target result based on the confidence information for the target result.

The method may further include discarding the target result in response to the confidence information for the target result being equal to or less than a reference.

The method may further include, based on the confidence information for the target result, requesting to retake the medical image input to the target artificial intelligence model or recommending an imaging method.

Some embodiments provide a computing apparatus including a processor configured to, when a target artificial intelligence model receiving a medical image outputs a target result, estimate confidence information for the target result based on at least one impact factor that affects the target result, and provide a user interface screen with the target result and the confidence information.

The processor may be further configured to receive the medical image, obtain the at least one impact factor determined based on a characteristic of the target artificial intelligence model and/or a characteristic of the medical image, and estimate the confidence information for the target result based on the at least one impact factor.

The processor may be further configured to perform at least one of correcting the confidence information for the target result based on the target result, and providing the corrected confidence information as final confidence information for the target result, correcting the target result based on the confidence information for the target result and providing the corrected target result, based on the confidence information for the target result, requesting to retake the medical image or recommending an imaging method, and discarding the target result in response to the confidence information for the target result being equal to or less than a reference.

According to some embodiments, it is possible to provide confidence information for a result of an artificial intelligence model in a form that is easily understandable to users.

According to some embodiments, it is possible to visually provide confidence information for the result along with the result of the artificial intelligence model.

### [Description of the Drawings]

FIG. 1 is a drawing illustrating a computing apparatus according to some embodiments.
FIG. 2 is a drawing illustrating an interworking environment of a computing apparatus according to some embodiments.
FIG. 3 is a diagram illustrating a method of providing confidence information for a target result according to some embodiments.
FIG. 4 is a flowchart of a method of providing confidence information for a target result according to some embodiments.
FIG. 5 is a drawing illustrating an example of an interface screen that provides confidence information for a target result of a medical image according to some embodiments.
FIG. 6 is a block diagram illustrating a hardware structure of the computing apparatus according to some embodiments.

### [Mode for Invention]

In the following detailed description, some embodiments of the present disclosure have been shown and described, but the inventive concepts are not limited thereto. Those of ordinary skill in the art would realize that the described embodiments may be modified in various different ways without departing from the spirit or scope of the inventive concepts described herein. Further, the drawings and description are to be regarded as illustrative in nature and not restrictive. Like reference numerals designate like elements throughout the specification.

In addition,an expression such as "have," "comprise," or "include" indicates existence of a corresponding feature (e.g., elements such as a numerical value, function, operation, or component) and does not exclude the presence of another feature. Furthermore, one or more of elements disclosed below may include or be implemented in one or more processing circuitries such as hardware including logic circuits, a hardware/software combination such as a processor executing software, or a combination thereof

An apparatus described in the present disclosure is a computing apparatus configured and connected so that at least one processor performs operations of the present disclosure by executing instructions. A computer program may include instructions that cause a processor to execute the operations of the present disclosure and may be stored on a non-transitory computer readable storage medium. The computer program may be downloaded through a network or sold as a product.

Medical images of the present disclosure may include images of various parts of body imaged with various modalities. For example, the modalities of the medical images may include x-rays, magnetic resonance imaging (MRI), ultrasound, computed tomography (CT), mammography (MMG), or digital breast tomosynthesis (DBT).

A user of the present disclosure may be a medical expert such as a physician, a nurse, a clinical pathologist, a sonographer, a medical imaging professional, as well as an ordinary person such as a patient or a guardian, but is not limited to these examples.

An artificial intelligence (AI) model of the present disclosure may be a machine learning model designed to learn at least one task, which may be implemented as a computer program executed by a processor. The AI model may be implemented as a computer program executed on a computing apparatus, downloaded through a network, or sold as a product. Alternatively, the AI model may be networked with a variety of devices.

A task that an AI model learns may refer to the task to be solved by machine learning, or the task to be performed by machine learning. For example, assuming that recognition, classification, and prediction are performed on a medical image, the recognition, classification, and prediction may correspond to different tasks.

In the present disclosure, a result of an AI model may refer to a result provided by the AI model that has learned the task based on an input. The result of the AI model may refer to at least one of various inference or prediction results analyzed from the input, including, for example, an abnormality score, a computer aided detection CAD result, and a disease prediction (e.g., cancer prediction).

The present disclosure may provide confidence information on the result of the AI model, and the confidence information may include a confidence score. The confidence score may represent a metric that indicates how accurate an AI model's performance on a task is. Thus, the confidence information on the result of the AI model in the present disclosure may be referred to as a "confidence measure score" to distinguish it from an AI model's confidence score on the task.

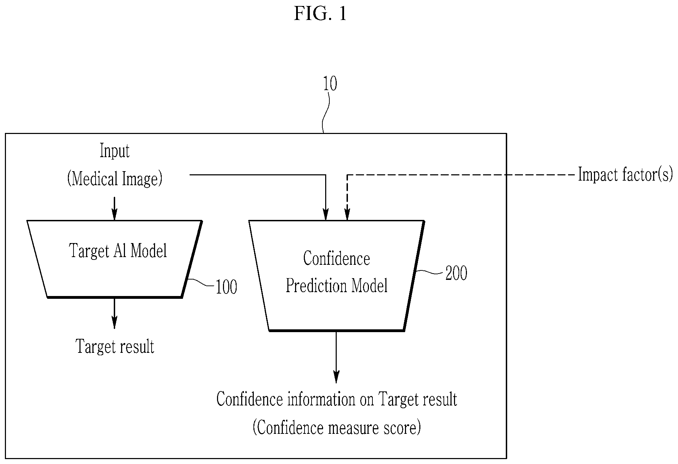

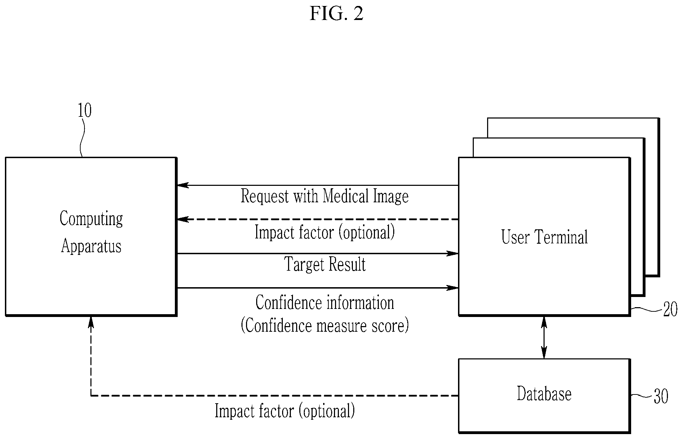

FIG. 1 is a drawing illustrating a computing apparatus according to some embodiments, and FIG. 2 is a drawing illustrating an interworking environment of a computing apparatus according to some embodiments.

Referring to FIG. 1, a computing apparatus 10 operated by at least one processor provides confidence information for a result of a target AI model 100. Herein, the confidence information for the result of the target AI model 100 may be estimated by using at least one impact factor that affects the result of the target AI model 100. The result of the target AI model 100 may simply be referred to as a "target result". The confidence information for the target result may be provided per target result, for example, the confidence information may be provided per lesion or per case.

A typical AI model may determine a confidence score based on the likelihood of the result provided by the AI model. For example, an AI model that detects lesions in medical images may determine the confidence score for the lesion detection result based on the likelihood of the result provided by the AI model. However, it may be difficult for users to clearly interpret a meaning of the likelihood and to intuitively understand a relationship between the likelihood and confidence score for the result.

Thus, the computing apparatus 10 according to various embodiments of the present disclosure may provide confidence information for a target result to a user in an understandable form. The computing apparatus 10 may predict an abnormality score for at least some tissue in a medical image as a target result, and represent a confidence score for the target result as a numerical value. For example, the computing apparatus 10 may estimate an abnormality score for a subject to be 71 and estimate a confidence score for the abnormality score of 71 to be 98%. The computing apparatus 10 may correct the target result based on the confidence information for the target result and provide the corrected target result. Alternatively, the computing apparatus 10 may correct the confidence information for the target result based on the target result and provide the corrected confidence information as final confidence information. Moreover, the computing apparatus 10 may use the confidence information for the target result to improve the target AI model 100.

When the confidence information for the target result is equal to or less than a reference, the computing apparatus 10 may discard the target result, and recommend that the user retake the medical image, or recommend an imaging method to obtain the target result with higher confidence information. The computing apparatus 10 may generate a report for the user, based on the confidence information for the target result. As such, the confidence information for the target result estimated by the computing apparatus 10 may be provided to the user together with the target result, be used to correct the target result, be used as an indicator to recommend to retake the medical image and/or the imaging method, or be used as an indicator to discard the target result analyzed by the target AI model 100. In addition, the confidence information for the target result may be used as a performance improvement index for the target AI model.

The computing apparatus 10 may include the target AI model 100, and a confidence prediction model 200 that provides the confidence information for the result of the target AI model 100.

For simplicity, the following operations are described as being performed on a single computing apparatus 10. However, the present disclosure is not limited to these examples, and depending on implementation, at least one of the operations described below may be performed on a plurality of computing apparatus and/or user terminals. For example, each of the target AI model 100 and the confidence prediction model 200 may be executed on separate computing apparatuses, and a user terminal may receive output results from the target AI model 100 and the confidence prediction model 200 and display the received output results on its screen.

The target AI model 100 is a model that has been trained on at least one task and performs a task for an input to output a target result. During a training process, the target AI model 100 may learn relationships between inputs and outputs, and output a result inferred from a new input. While the input to the target AI model 100 may be various types of data, for the purposes of description, it is assumed to be a medical image. For example, the target AI model 100 may be a model trained to receive a medical image as an input and to detect a lesion in the medical image or infer medical diagnostic information or treatment information. The medical information may include various medical inference/prediction results, such as an abnormality score of a lesion and a disease occurrence risk predicted based on the lesion.

The confidence prediction model 200 may obtain at least one impact factor that affects the target result, and provide confidence information for the target result based on the impact factor. The impact factor may be determined from the target AI model 100 and a characteristic of an input (e.g., a medical image) to the target AI model 100.

The confidence prediction model 200 may include a model that has learned a relationship between at least one impact factor associated with the medical image for training and a confidence score of a target result inferred from the corresponding medical image for training. Here, the target result inferred from the medical image for training may be an output of the target AI model 100. Further, the confidence prediction model 200 may include a model trained to infer at least one impact factor from the input medical image.

In an example, the confidence prediction model 200 may interwork with a picture archiving and communication system (PACS), an electronic medical record (EMR), and/or an electronic health record (EHR) to obtain various clinical information related to a patient. The confidence prediction model 200 may extract or infer the impact factor from the various clinical information related to the patient.

In another example, the confidence prediction model 200 may extract the impact factor from additional information (e.g., metadata) included in the input image. For example, when the input image conforms to the digital imaging and communications in medicine (DICOM) standard, the confidence prediction model 200 may extract at least one impact factor from imaging information, including age, gender, and an imaging method, recorded in a header of DICOM data, .

In yet another example, the confidence prediction model 200 may infer from the input image the impact factor including, for example, tissue density, presence of an object in an image, image quality, a type of lesion (e.g., soft tissue or calcification), or a change in size of a lesion in an image analyzed by the AI through a comparison with a past image. For this purpose, the confidence prediction model 200 may include an AI model trained to infer at least one impact factor from the input image.

In addition, the confidence prediction model 200 may interwork with an external server/database to obtain the impact factors. Alternatively, the confidence prediction model 200 may receive the impact factors from the user input.

The confidence prediction model 200 may predict confidence information for the target result using the impact factors vectorized in a specified manner. For example, a numerical impact factor may be quantified as a value between 0 and 1, and a categorical impact vector may be quantified using one-hot encoding. For example, based on breast density inferred from the input image, a value of a density impact factor may be quantized as 1 for dense and 0 otherwise, or as a value between 0 and 1. Alternatively, the impact factor obtained from metadata, an external server/database, or a user input may be quantified as 0 or 1 because it is relatively precise values, and the impact factor inferred by the confidence prediction model 200 may be quantified as a value between 0 and 1 based on an inference probability.

The confidence prediction model 200 may predict confidence information for each result of the target AI model 100. Here, the confidence information for the result of the target AI model 100 may simply be referred to as a confidence measure score. The confidence prediction model 200 may be implemented as an AI model trained to estimate confidence information from at least one impact factor, or as a statistical model that calculates the confidence information from the at least one impact factor. For example, the statistical model may include statistical information for at least one impact factor that affects the target result (e.g., an abnormality score).

The impact factors that affect the target result of the target AI model may be determined from among candidate factors, such as a task-related medical factor of the target AI model 100, an input image-related factor, a disease-related factor, a patient-related demographic factor, and a patient characteristic-related factor. For example, when the AI model receives a medical image and outputs a target result such as a prediction/inference of the medical image, the confidence prediction model 200 may use at least a part of factors in Table 1 as impact factors that affect the result of the AI model.

**Table 1**

| Impact factor | Explanation and example |
|---|---|
| Task-related medical factor | Breast density related to lesion detection task from mammogram images |
| Input image-related factor | Imaging details (e.g., imaging method (e.g., posterior-anterior (PA) or anterior-posterior (AP)), imaging posture, imaging position, or imaging equipment) |
| | Presence of objects (e.g., medical device, extrinsic material, button, marker, tube, or pacemaker) in images |
| | Image quality |
| | Tissue density |
| | Lesion type (e.g., soft tissue, calcification, or extrinsic material) |
| | Change in lesion size |
| Disease-related factor | Type of disease, accuracy of disease detection, or the like |
| Patient-related demographic factor, and patient characteristic-related factor | Gender, age, race, geographic characteristic, or the like |

The confidence prediction model 200 may use the task-related medical factor as the impact factor. For example, when the input image is a mammogram image, it is known that higher breast density makes it more difficult for an AI model to accurately detect lesions and leads to a higher probability of false positive (FP). As such, since density in relation to reading difficulty is an impact factor that affects the result, the confidence prediction model 200 may estimate confidence information for the result of the target AI model 100 based on tissue density inferred from the image. In this case, the confidence prediction model 200 may receive the image input to the target AI model 100, infer the density of the image, and estimate the confidence information for the result of the target AI model 100 based on the inferred density.

The confidence prediction model 200 may use various input image-related factors as the impact factors. For example, imaging details of a medical image, presence of an object in the image, image quality, tissue density, lesion type, change in lesion size, or the like may be used as the impact factors. The input image-related factors may be extracted from additional information (e.g., a header in DICOM data and metadata in the medical image) of the medical image or inferred from the medical image.

The imaging details may include, for example, an imaging method e.g., PA or AP), imaging posture, an imaging position, or imaging equipment. The confidence prediction model 200 may extract the imaging details from additional information included in the input image, or obtain the imaging details from an external server/database. The confidence prediction model 200 may estimate the confidence information for the result of the target AI model 100 based on the imaging details of the image input to the target AI model 100. For example, when dealing with chest X-ray images, it is known that PA images are more difficult to interpret than AP images. The AI model also suffers from such reading difficulty. Therefore, when a chest X-ray image is input to the AI model, information of whether the chest X-ray image is a PA image or an AP image may be used as an impact factor. Further, the type and characteristics of the imaging equipment may affect the result of the AI model. For example, an x-ray machine may use different imaging methods such as a film method, a computed radiography (CR) method, or a digital radiography (DR) method, and the chosen imaging method may affect the result of the AI model. In addition, the manufacturing company or product version of the imaging equipment may affect the result of the AI model.

The object present in the image may include, for example, a medical device, an extrinsic material, a button, a marker, a tube, or a pacemaker. For example, it is known that when the image contains the object that is not part of a patient's biological tissue, the reading confidence tends to be low. Therefore, the confidence prediction model 200 may receive the image input to the target AI model 100, extract the object in the image, and estimate confidence information for the result of the target AI model 100 based on the extracted object.

The image quality may also be used as the impact factor that affects the result of the target AI model. For example, it is known that poor image quality, such as presence of artifact in the image or low resolution of the image, results in the decreased reading confidence. Therefore, the confidence prediction model 200 may receive the image input to the target AI model 100, obtain the image quality, and estimate the confidence information for the result of the target AI model 100 based on the obtained image quality.

Various information inferred from the image may be used as the impact factors that affect the result of the target AI model. As described in the relationship between the breast density and the reading difficulty, the tissue density inferred from the image may be used to estimate the confidence of the target result. The type of lesion, such as soft tissue or calcification lesion, may be inferred from the image, and may be used as the impact factor that affects the result of the target AI model. In addition, the change in lesion size may be inferred by analyzing a past image of a patient related to the analysis target image, and may be used as the impact factor that affects the result of the target AI model.

The disease-related factor may be used as the impact factor. For the AI model that predicts a disease, the accuracy of disease detection may vary depending on an amount of disease-related training data and the characteristic of the disease itself. Therefore, the disease-related factor, such as the type of disease or the accuracy of disease detection, may affect the result of the AI model. The confidence prediction model 200 may estimate confidence information for the result of the target AI model 100 based on the disease-related factor detected by the target AI model 100.

In addition, the patient-related demographic factor or the patient characteristic-related factor may be used as the impact factor. For example, the patient-related demographic factors, such as gender, age, race, or geographic characteristic, may affect the result of the AI model. Furthermore, the patient characteristic-related factor, such as patient personal information, may affect the result of the AI model. The confidence prediction model 200 may extract the patient-related demographic factor or the patient characteristic-related factor from the additional information included in the input image, or obtain the patient-related demographic factor or the patient characteristic-related factor from an external server/database. Alternatively, the confidence prediction model 200 may also infer the patient-related demographic factor or the patient characteristic-related factor from the input image.

Referring to FIG. 2, the computing apparatus 10 may be implemented as a standalone device or to interwork with another device. For example, the computing apparatus 10 may be implemented to interwork with a plurality of user terminals 20. In addition, the computing apparatus 10 may interwork with various databases 30 within a healthcare organization, including, for example, a picture archiving and communication system (PACS), an electronic medical record (EMR), or an electronic health record (EHR), to access various clinical information of a patient. The user terminal 20 may be connected to the computing apparatus 10 and the database 30 to provide a user interface screen that displays required information on a screen. The user terminal 20 may display information provided by the computing apparatus 10 on a dedicated viewer.

The computing apparatus 10 may be a server device, and the user terminal 20 may be a client terminal installed within the healthcare organization. The computing apparatus 10 and the user terminal 20 may be interconnected through a network. The computing apparatus 10 may be a local server connected to a network within a particular healthcare organization. The computing apparatus 10 may be implemented as a cloud server, enabling interconnection with terminals (e.g., medical staff terminals) from a plurality of medical organizations with access rights. The computing apparatus 10 may be implemented as a cloud server and may interwork with a patient's personal terminal having the access right.

The computing apparatus 10 may receive a request from the user terminal 20 to analyze a medical image, and may provide a response to the user terminal 20, including a target result and confidence information (confidence measure score) for the target result. The computing apparatus 10 may obtain the impact factors from the user terminal 20 or the database 30. In cases where the medical image is stored in the database 30, the user terminal 20 may transmit the medical image retrieved from the database 30 to the computing apparatus 10, or the computing apparatus 10 may retrieve the medical image requested by the user terminal 20 from the database 30.

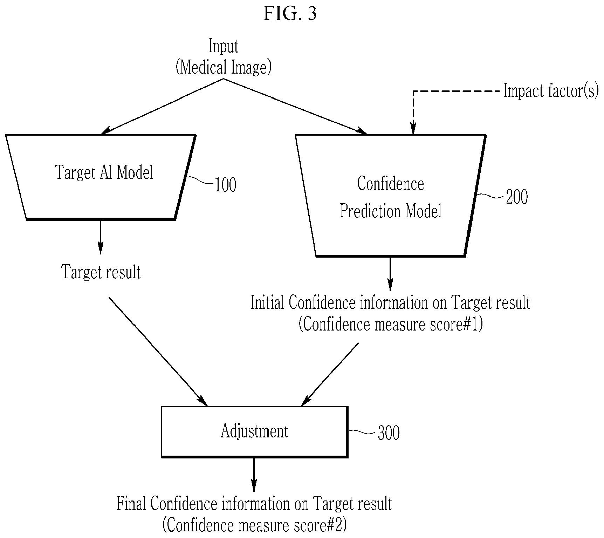

FIG. 3 is a diagram illustrating a method of providing confidence information for a target result according to some embodiments.

Referring to FIG. 3, a confidence prediction model 200 may obtain at least one impact factor that affects a result of a target AI model 100, and may provide confidence information for a target result based on the impact factor.

The impact factors for the target AI model may be determined from among candidate factors, including a task-related medical factor, an input image-related factor, a disease-related factor, a patient-related demographic factor, and a patient characteristic-related factors. For example, the confidence prediction model 200 may extract the impact factors from additional information (metadata) included in an input image. The confidence prediction model 200 may infer the required impact factors from the input image. The confidence prediction model 200 may interwork with an external server or database to obtain the impact factors. Alternatively, the confidence prediction model 200 may receive the impact factors as an input from a user.

As shown in FIG. 3, a computing apparatus 10 according to some embodiments may provide initial confidence information output from the confidence prediction model 200 as the confidence information for the target result, and may correct the initial confidence information to provide more accurate final confidence information.

The computing apparatus 10 may calculate the final confidence information using an adjustment module 300. The computing apparatus 10 may correct the initial confidence information based on the target result of the target AI model 100. The adjustment module 300 may correct the initial confidence information based on the target result, for example, using a weighted sum operation. Alternatively, the adjustment module 300 may be implemented as an AI model that has learned an input-output relationship, or as a statistical model that includes statistical information of the input-output relationship.

According to some embodiments different from the embodiments illustrated in FIG. 3, the computing apparatus 10 may provide the target result, that is, an initial target result, output from the target AI model 100, as output information, and may correct the initial target result to provide a more accurate final target result.

The computing apparatus 10 may calculate a final target result using the adjustment module 300. The computing apparatus 10 may correct the initial target result based on the confidence information provided by the confidence prediction model 200. The adjustment module 300 may use the confidence information to correct the initial target result, for example, using a weighted sum operation. Alternatively, the adjustment module 300 may be implemented as an AI model that has learned the input-output relationship, or as a statistical model that includes statistical information of the input-output relationship.

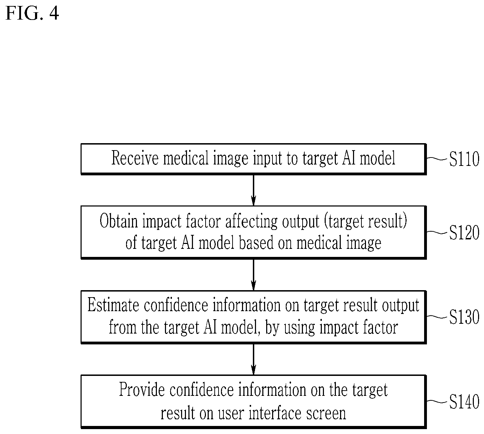

FIG. 4 is a flowchart of a method of providing confidence information for a target result according to some embodiments.

Referring to FIG. 4, a computing apparatus 10 receives a medical image input to a target AI model 100 in S 110. The target AI model 100 may receive the medical image from the computing apparatus 10, an external device (e.g., a user terminal or database), or a memory within the computing apparatus 10.

The computing apparatus 10 obtains at least one impact factor that affects an output (target result) of the target AI model 100 based on the medical image in S 120. The computing apparatus 10 may determine a type of impact factor based on a characteristic of the target AI model 100 and/or a characteristic of an input (e.g., medical image). For example, the impact factor may be determined from among candidate factors including, for example, a task-related medical factor (e.g., breast density) of the target AI model 100, an input image-related factor (e.g., imaging details, presence of an object in the image, image quality, tissue density, lesion type, and change in lesion size), a disease-related factor (e.g., disease type and accuracy of disease detection), a patient-related demographic factor, and a patient characteristic-related factors. The computing apparatus 10 may extract at least one impact factor from additional information (metadata) included in the input of the target AI model 100. The computing apparatus 10 may infer the impact factor including, for example, the object in the image, the image quality, the tissue density, the lesion type, or the change in lesion size, from the input to the target AI model 100. The computing apparatus 10 may interwork with an external server or database to obtain the impact factor. Alternatively, the computing apparatus 10 may receive the impact factor as an input from a user. The computing apparatus 10 may quantify the obtained impact factor in a specified manner. For example, the computing apparatus 10 may quantify the impact factor as a value between 0 and 1.

The computing apparatus 10 estimates confidence information for the target result output from the target AI model 100 based on the obtained at least one impact factor in S130. The computing apparatus 10 may estimate the confidence information for the target result using a confidence prediction model 200, which is implemented as an AI model trained to estimate confidence information from impact factor, or as a statistical model that calculates confidence information from impact factors.

The computing apparatus 10 provides the confidence information for the target result to a user interface screen in S 140. The computing apparatus 10 may provide the confidence information for the target result on a screen of a user terminal 20. The computing apparatus 10 may correct the confidence information for the target result based on the target result to provide more accurate final confidence information. The computing apparatus 10 may correct the target result based on the confidence information for the target result. The computing apparatus 10 may provide the confidence information for the target result along with the target result. The confidence information for the target result may be provided per target result. For example, the confidence information may be provided per lesion or per case.

On the other hand, the computing apparatus 10 may discard the target result when the confidence information for the target result is equal to or less than a reference. Depending on the confidence information for the target result, the computing apparatus may prompt a user for a new input to obtain a more accurate target result. For example, when the confidence information for the target result is equal to or less than the reference, the computing apparatus may request the user to retake a medical image or recommend an imaging method to obtain a target result with higher confidence information.

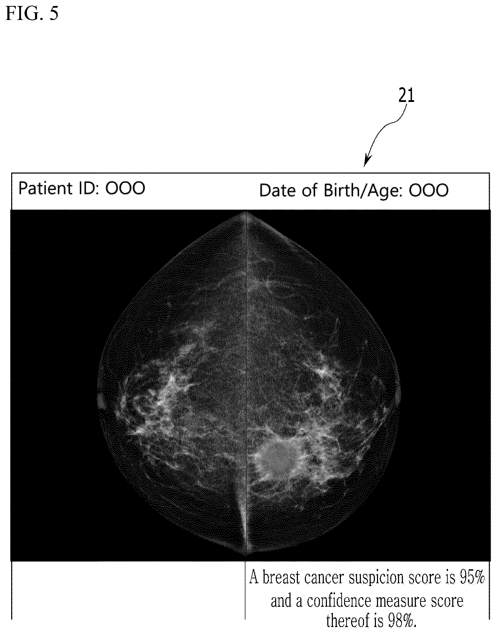

FIG. 5 is a drawing illustrating an example of an interface screen that provides confidence information for a target result of a medical image according to some embodiments.

Referring to FIG. 5, it is assumed that a target AI model 100 outputs an abnormality score of a mammogram image to a user terminal 20 as a target result.

During or after the target AI model 100 infers or predicts the abnormality score of the mammogram image, a computing apparatus 10 may collect at least one impact factor that affects image reading of the target AI model 100. The computing apparatus 10 may determine a density of the mammogram image as the impact factor. The computing apparatus 10 may estimate a confidence measure score for the abnormality score output by the target AI model 100 based on the density obtained from the mammogram image. In addition to the density, the computing apparatus 10 may estimate a confidence information for the abnormality score based on at least one impact factor determined from among candidate factors including an input image-related factor (e.g., imaging details, object in the image, image quality, lesion type, and change in lesion size), a disease-related factor (e.g., type of disease, and accuracy of disease detection), a patient-related demographic factor, and a patient characteristic-related factor.

A user terminal 20 displays information provided by the computing apparatus 10 on a user interface screen 21. For example, the screen 21 may display a heat map showing a nodule detected in the mammogram image. Further, the screen 21 may display a breast cancer suspicion score, which may be represented by an abnormality score of the nodule, and a confidence measure score for the breast cancer suspicion score. For example, the screen 21 may display that the breast cancer suspicion score (probability) is 95% and that the confidence measure score for the result of 95% is 98%.

A user may make final decision regarding presence of an abnormal lesion and/or a subsequent course of action, based on an analysis result of the AI model. In this case, the computing apparatus 10 according to some embodiments may provide the user with the result analyzed by the AI model and the confidence information for the analysis result, which may help the user to consider the target result analyzed by the AI model and make the final decision. In addition, the computing apparatus 10 according to some embodiments may improve the accuracy of the analysis result (target result) of the AI model by reflecting the estimated confidence information into the analysis result of the AI model.

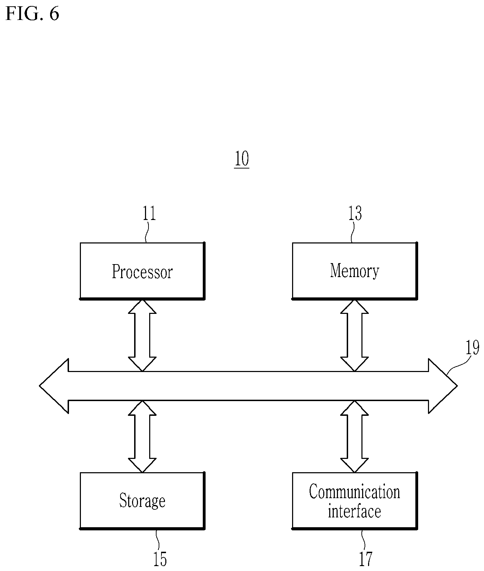

FIG. 6 is a block diagram illustrating a hardware structure of a computing apparatus according to some embodiments.

Referring to FIG. 6, a computing apparatus 10 may include one or more processors 11, a memory 13 configured to load one or more computer programs executed by the processors 11, a storage device 15 configured to the computer programs and various data, a communication interface 17, and a bus 19 connecting them. In addition, the computing apparatus 10 may further include various other components. The computer program may include one or more instructions that, when the computer program is loaded into the memory 13, cause the processor 11 to perform a method or operations according to various embodiments of the present disclosure. That is, by executing the one or more instructions, the processor 11 may perform the method or operations according to various embodiments of the present disclosure. The computer program is a set of computer-readable instructions grouped by function and executed by a processor. The computer program may include a target AI model trained to infer medical diagnostic information or treatment information from an input image, a confidence prediction model that provides confidence information for an inference result of the target AI model, and/or an adjustment model that corrects an initial confidence information and/or an initial target result.

The processor 11 controls overall operations of each component of the computing apparatus 10. The processor 11 may include at least one of a central processing unit (CPU), a microprocessor unit (MPU), a micro controller unit (MCU), a graphics processing unit (GPU), or any other form of processor known in the art of the present disclosure. Further, the processor 11 may perform operations on at least one application or computer program for executing the method or operations according to various embodiments of the present disclosure.

The memory 13 stores various data, instructions, and/or information. The memory 13 may load one or more computer programs from the storage device 15 to perform the method or operations according to various embodiments of the present disclosure. The memory 13 may be implemented as a volatile memory such as RAM, but the technical scope of the present disclosure is not limited thereto.

The storage device 15 may non-temporarily store the computer program. The storage 15 may include a non-volatile memory, such as a read only memory (ROM), an erasable programmable ROM (EPROM), an electrically erasable programmable ROM (EEPROM), or a flash memory, a hard disk, a removable disk, or any other form of computer-readable recording medium known in the art of the present disclosure.

The communication interface 17 supports wired and wireless Internet communication of the computing apparatus 10. The communication interface 17 may further support a variety of communication methods other than the Internet communication. The communication interface 17 may be configured to include communication modules known in the art of the present disclosure.

The bus 19 provides communication functions between the components of the computing apparatus 10. The bus 19 may be implemented as various types of buses, such as an address bus, a data bus, and a control bus.

Embodiments of the present disclosure described above are not only implemented using the apparatus and method, but may also be implemented using at least one program that realizes functions described in the embodiments of the present disclosure, or using a recording medium on which the at least one programs is recorded.

While embodiments have been described in connection with what is presently considered to be practical embodiments, it is to be understood that example embodiments are not limited to the disclosed embodiments. On the contrary, it is intended to cover various modifications and equivalent arrangements included within the spirit and scope of the appended claims.

## Claims

1. A computing apparatus operated by at least one processor, the computing apparatus comprising:
a target artificial intelligence model configured to learn at least one task, and perform a task for an input medical image to output a target result; and
a confidence prediction model configured to obtain at least one impact factor that affects the target result based on the input medical image, and estimate confidence information for the target result based on the impact factor.

2. The computing apparatus of claim 1, wherein the at least one impact factor is determined based on a characteristic of the target artificial intelligence model and/or a characteristic of the input medical image.

3. The computing apparatus of claim 1, wherein the at least one impact factor comprises at least one of
a task-related medical factor of the target artificial intelligence model;
an input image-related factor of the target artificial intelligence model;
a disease-related factor detected by the target artificial intelligence model;
a patient-related demographic factor; and
a patient characteristic-related factor.

4. The computing apparatus of claim 1, wherein the at least one impact factor is:
extracted from additional information of the input medical image;
inferred from the input medical image;
obtained from an external server or database; or
input from a user.

5. The computing apparatus of claim 4, wherein the at least one impact factor extracted from the additional information of the input medical image comprises at least one of age, gender, and imaging details including an imaging method, and
wherein the at least one impact factor inferred from the input medical image comprises at least one of tissue density, presence of an object in the input medical image, a lesion type, and a change in lesion size.

6. The computing apparatus of claim 1, wherein the target artificial intelligence model comprises a model trained to detect a lesion from a medical image or to infer medical diagnostic information or treatment information.

7. The computing apparatus of claim 1, wherein the confidence prediction model comprises a model that has learned a relationship between at least one impact factor associated with a medical image for training and a confidence score for a target result inferred from the medical image for training.

8. The computing apparatus of claim 1, wherein the confidence information for the target result is:
provided together with the target result;
used for correction of the target result;
used as an indicator for recommending to retake the input medical image and/or for recommending an imaging method; or
used as an indicator for discarding the target result output from the target artificial intelligence model.

9. A method of operating a computing apparatus operated by at least one processor, the method comprising:
receiving a medical image input to a target artificial intelligence model;
obtaining, based on the medical image, at least one impact factor that affects a target result output from the target artificial intelligence model; and
estimating confidence information for the target result based on the at least one impact factor.

10. The method of claim 9, wherein the obtaining the at least one impact factor comprises:
extracting the at least one impact factor from additional information of the medical image;
inferring the at least one impact factor from the medical image;
obtaining the at least one impact factor from an external server/database; or
receiving the at least one impact factor from a user.

11. The method of claim 10, wherein the at least one impact factor extracted from the additional information of the medical image comprises at least one of age, gender, and imaging details including an imaging method, and
wherein the at least one impact factor inferred from the medical image comprises at least one of tissue density, presence of an object in the medical image, a lesion type, and a change in lesion size.

12. The method of claim 9, wherein the obtaining the at least one impact factor comprises:
in response to the medical image being a mammogram image, determining density inferred from the mammogram image as the at least one impact factor; or
in response to the medical image being a chest X-ray image, determining posterior anterior (PA) or anterior-posterior (AP) information extracted from additional information of the chest X-ray image as the at least one impact factor.

13. The method of claim 9, further comprising:
correcting confidence information for the target result based on the target result; and
providing the corrected confidence information as final confidence information for the target result.

14. The method of claim 9, further comprising
providing the confidence information together with the target result.

15. The method of claim 9, further comprising
correcting the target result based on the confidence information for the target result.

16. The method of claim 9, further comprising
discarding the target result in response to the confidence information for the target result being equal to or less than a reference.

17. The method of claim 9, further comprising
based on the confidence information for the target result, requesting to retake the medical image input to the target artificial intelligence model or recommending an imaging method.

18. A computing apparatus comprising:
a processor configured to:
when a target artificial intelligence model receiving a medical image outputs a target result, estimate confidence information for the target result based on at least one impact factor that affects the target result; and
provide a user interface screen with the target result and the confidence information.

19. The computing apparatus of claim 18, wherein the processor is further configured to:
receive the medical image;
obtain the at least one impact factor determined based on a characteristic of the target artificial intelligence model and/or a characteristic of the medical image; and
estimate the confidence information for the target result based on the at least one impact factor.

20. The computing apparatus of claim 19, wherein the processor is further configured to perform at least one of:
correcting the confidence information for the target result based on the target result, and providing the corrected confidence information as final confidence information for the target result;
correcting the target result based on the confidence information for the target result and providing the corrected target result;
based on the confidence information for the target result, requesting to retake the medical image or recommending an imaging method; or
discarding the target result in response to the confidence information for the target result being equal to or less than a reference.
